# EUROPEAN PATENT SPECIFICATION

(11) **EP 0 729 901 B1**
(45) Date of publication and mention of the grant of the patent: **12.07.2000**
(21) Application number: 95104281.1
(22) Date of filing: 23.03.1995
(51) Int. Cl.: B65D 77/22, B65D 51/16

(54) **Package or cap having a venting system with draining means**
Packung oder Kappe mit einem, mit einer Flüssigkeitsabzugsvorrichtung versehenen, Entlüftungssystem
Emballage ou fermeture muni d'un système de mise à l'air avec moyens de drainage

(43) Date of publication of application: 04.09.1996
(73) Proprietor: THE PROCTER & GAMBLE COMPANY, Cincinnati, Ohio 45202 (US)
(72) Inventor: Lake, Kirk Wallace, B-1933 Sterrebeek (BE); van den Branden, Bruno, B-1980 Eppegem (BE); Rogers, Neil John, B-1020 Brussel (BE)
(74) Representative: Engisch, Gautier

(56) References cited:
- EP-A- 0 002 155
- EP-A- 0 024 310
- WO-A-94/26614
- US-A- 1 389 659
- US-A- 4 765 499

## Description

### Field of the invention

The present invention relates to a package or a cap for a package which enables viscous, liquid products to be vented. This package or cap improves the drainage of product away from a venting means of said package or cap.

### Background of the invention

The problem of package deformation in response to pressure differences existing between the inside of a closed package and the ambient pressure is well known in the packaging industry. Such package deformation may be non-recoverable for certain package materials, like some plastics or metals. Thin-walled, partially flexible packages are particularly sensitive to the problem.

There are a number of possible factors which may lead to the existence of the pressure differences between the interior and the exterior of the package mentioned above. The content of the package may, for example, be chemically unstable or may be subject to reaction with gases which may exist in the head space of the package, or alternatively, in certain specific circumstances, may react with the package material itself. Any chemical reactions involving the liquid contents may lead to either production of gases, and hence to overpressure in the package, or to the absorption of any head space gases thereby causing underpressure in the package.

Pressure differences between the pressure inside the container and the ambient atmospheric pressure may also occur when the temperature during the filling and sealing of the container is significantly different from external temperature during shipment, transportation and storage. Another possibility of a pressure difference may be caused by a different ambient pressure at the filling of the container from another ambient pressure at a different geographical location.

The prior art has proposed several solutions using valve systems which avoid pressure differences between the interior and the exterior of the package. Proposed solutions also relate to various venting caps which allow pressure generated inside the package to be released by escape of gas. For example, FR-A-2 259 026, US-4 136 796 and DE-A-2 509 258 disclose self-venting closures comprising a gas-permeable membrane covering an orifice to the exterior. Said membranes are made of a material which is impermeable to liquids, but permeable to gases. Therefore, packages may comprise apertures to release gas to the exterior without losing their leak-tightness. Another example is EP-A-593 840 which discloses containers for containing liquids which generate pressure, said container being made of a thermoplastic material comprising a network of micro-channels. This network of microchannels is permeable to gases, but not to liquids.

Another example is US.4.765.499 (von Reis et Al.) filed 29 December 1987. It discloses a venting closure for containers having a pour opening, said venting closure comprising a liner with a portion which is hydrophobic but gas-permeable, so as to create a venting means. The rest of the liner, especially the periphery which is in contact with the pour opening of the container is non-hydrophobic, and non gas-permeable, so as to create a perfect seal when the container is closed with the cap.

We found that should liquid product contact these membranes, or the extremity of micro-channels, said membranes may lose at least part of their gas-permeability. Also, liquid products which are viscous or which have some affinity for these membranes may not drain away from said membrane back into the package. Therefore, should said membranes become contacted by such products, their venting performance is substantially lost for any part of said membrane covered by the product which has not drained away from said membrane. In this manner, it may happen that the package loses venting capacity. This loss of venting capacity results in a pressure difference between the exterior and the inside of said package which may deform said package.

Said product may splash onto said membrane as the filled package is agitated during shipment and transportation of the package. We found that the amount of splashes normally occurring during shipment and transportation are sufficient to completely interrupt the venting capacity of said package. Another means by which product may contact with the membrane is during an upside down storage of the package. We further found that other venting systems, like valves for example, may also suffer from a similar disadvantage.

It is therefore an object of the present invention to provide a package (10) for a liquid product, or a cap (10) for such a package which allows venting of said product by a venting means (20), and enabling the venting by draining said product away from said venting means once said product has come in contact with said venting means.

### Summary of the Invention

The present invention provides a package containing a liquid product according to claim 1. The present invention also provides a package (10) for a liquid product, or a cap (10) for such a package, according to claim 4. Said product has a viscosity of at least 5 cps measured when using a Brookfield viscosity meter at 60 rpm, spindle 3 and 20° Celsius. Said package or cap further enables the venting of said product by a venting means (20). Said venting means allows passage of gases between the interior and the exterior of said package when the pressure inside said package differs from the ambient pressure. Said venting means is further permeable to gases, but impermeable to said product. Said venting means comprises an outer surface (22). Said package or cap comprises a means (30) which enables said product to drain away from said outer surface of said venting means, once said product has come in contact with said outer surface of said venting means.

### Brief description of the figures

Figures 1a and 1b are cross sectional views of a package or a cap (partially shown) of the prior art comprising a venting means.

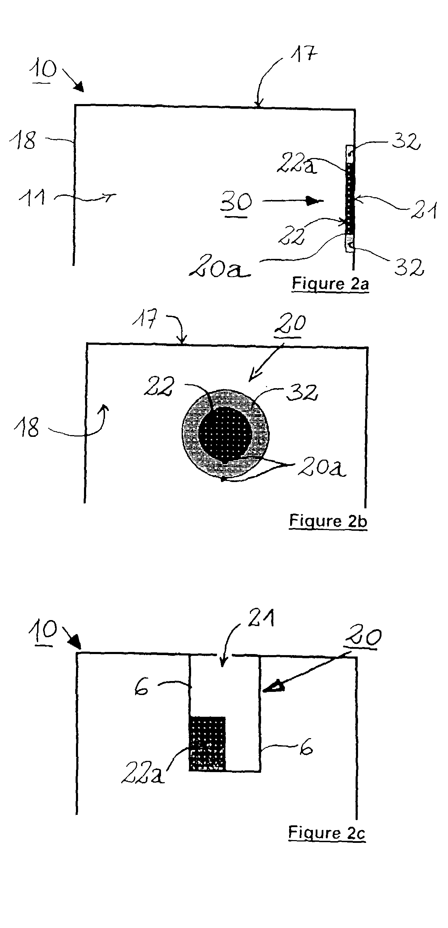

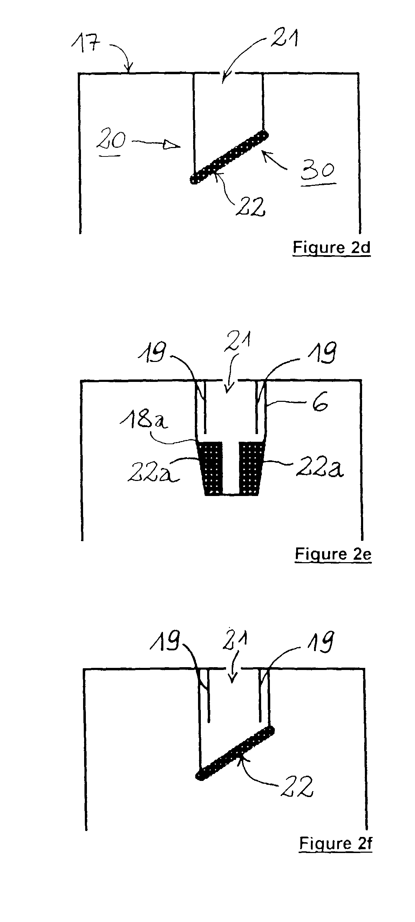

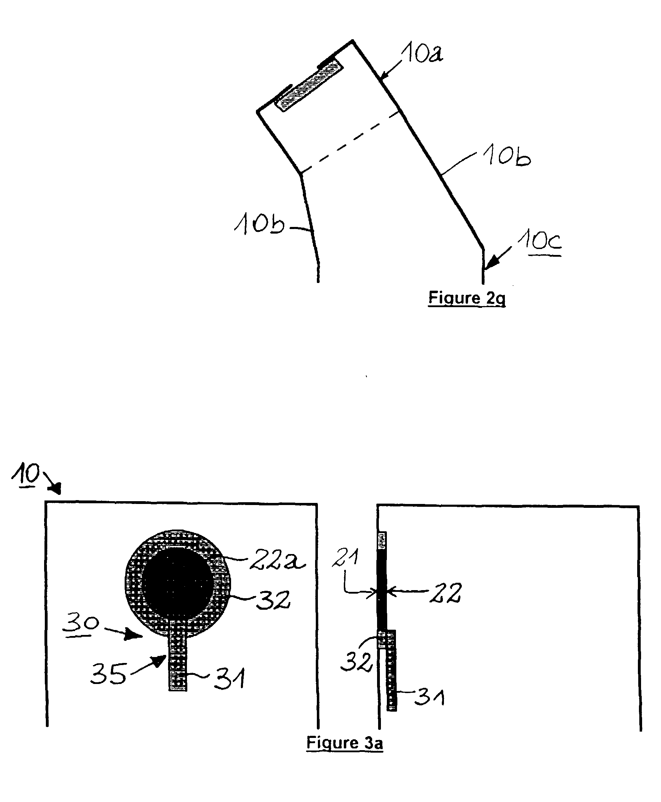

Figure 2a shows a cross sectional side view and Figure 2b shows its cross sectional front view of a package (partially shown) or of a cap in an embodiment which improves the drainage of product away from the venting means according to the present invention. Figures 2c to 2f show cross sectional views of a package (partially shown) or of a cap in other embodiments improving the drainage of product away from the venting means according to the present invention. Figure 2g illustrates an elevated side view of a package (partially shown) and its corresponding cap in an embodiment according to the present invention.

Figures 3a to 3f show cross sectional front and side views of possible draining means according to the present invention complementary to the embodiments of Figures 2.

Figures 4a to 4d illustrate cross sectional side views of possible draining means according to the present invention when the venting means is positioned horizontally in a package or a cap.

Figures 5a and 5b show cross sectional side views and bottom views of other possible draining means according to the present invention when the venting means is positioned horizontally in a package or a cap.

Figures 6a and 6b illustrate cross sectional side views of other possible draining means according to the present invention when the venting means is positioned horizontally in a package or a cap.

Figures 7a to 7d show cross sectional side views and bottom views of other possible draining means according to the present invention when the venting means is positioned horizontally in a package or a cap.

### Detailed description of the invention

As used herein, "vertical" is a position which is inclined perpendicularly to the plane on which said package stands in its upright position, e.g. said venting means or else may be located on a side wall of said package or cap. By "horizontal" it is intended a position parallel (no inclination) to the plane on which said package stands in its upright position, e.g. said venting means or else may be located parallel to the top wall of said package or cap. An "inclined position" is a position intermediate between the horizontal and vertical position.

In the following, the drawings may refer to a portion of a package as well as to a cap as well as to any structure attached to said package. Indeed, the present invention may be part of a cap only, whereby said cap may be then engaged to any package filled with gasifying liquid products. A cap of the screw-on/in or snap-on/in type, or a flip-top, push-pull or turret cap closures may be engagement means between said cap and said package.

In the following, Figure 2a will be described first as a package, then as a cap. In the first case, Figure 2a shows a cross sectional side view of a package, the package (10) (only partially shown) comprises a hollow body (11). Figure 2b is the corresponding cross sectional front view of said package. Said hollow body may comprise a top wall (17), a side wall (18) and a bottom wall (not shown in Figure 2a). Said hollow body is able to contain any liquid products. Preferably, said hollow body is flexible to an extend that it may deform in response to pressure differences arising between the inside of said package and the ambient pressure. Pouches made of thin plastic material, for example, are also encompassed by the present invention. Otherwise, suitable shapes of said package may include essentially cylindrical, tapered cylindrical, oval, square, rectangular or flat-oval.

Said hollow body should be suitable for leak-tight containment of liquid products having a viscosity of at least 5 cps, where this viscosity is measured using a Brookfield viscosity meter, spindle 3, 60 rpm at 20°C. In the following, liquid products encompass also pastes, creams, gels, emulsions and slurries. Said products may include, for example, household products such as detergents for laundry or dish washing, hard-surface and household cleaners, shampoos, bleaches, personal/beauty care products, creams and toothpastes. According to the present invention, said hollow body is able to contain liquid gasifying products. For example, gasifying products may be liquid laundry products comprising a bleach, particularly peroxygen bleach. Preferably said product has a viscosity of between 200 cps and 5000 cps, more preferably of between 500 cps and 1800 cps, most preferably of between 800 cps and 1600 cps, where this viscosity is measured using a Brookfield viscosity meter, spindle 3, 60 rpm at 20°C.

In case Figure 2a represents a cross sectional side view of a cap, the cap (10) comprises a top wall (17) and a side wall (18). As before, Figure 2b is the corresponding cross sectional front view of said package. Said cap can be engaged in a leak tight manner to the package described before. In another preferred embodiment of the present invention, said package or cap (10) may comprise a spout. Preferably, said package or cap is made of plastic, metal, paper, or combinations of these materials as layers, laminates or co-extrudates. The materials may be also recycled. Preferred materials for said hollow body include plastics such as polyethylene (high or low density), polyvinyl chloride, polyester, polyethylene terephthalate (=PET), extrudable PET, polypropylene, polycarbonate and nylon. These plastics may be used individually or be combined as co-extrudates, layers or laminates.

Another essential feature of said package or cap (10) comprises a venting means (20). Said venting means is able to equalize the pressure inside said package to the external atmospheric pressure. Consequently, said venting means is able to avoid overpressure as well as underpressure inside said package. Indeed, said venting means allows the escape of gases released from the contained product from the inside to the outside of said package, or vice versa. Said venting means is located in the upper portion of said package above the level of said contained product, when said package is in its upright position. Indeed, the gases causing the overpressure or underpressure accumulate typically in the upper region of the package. Therefore, the passage of gases to the exterior or interior is facilitated.

Preferably, said venting means comprises at least an orifice (21) and an outer surface (22). Said orifice connects the interior of said package with the exterior. Specifically, said orifice (21) allows the passage of gases from the interior to the exterior of said package, or vice versa, such that pressure inside said package is either maintained identical to the external atmospheric pressure or at a pressure at least below the pressure at which significant bottle deformation occurs. The dimension of said orifice should be suitable for said passage of gases. Preferably, said orifice is circular and has a diameter of at least 0.5 mm, preferably between 1 mm to 3 mm. The number of orifices can be chosen by a person skilled in the art to allow a sufficient amount of flow of the gases.

Said outer surface (22) is located around said orifice and between the content of said hollow body (11) and said orifice (21) in the interior of said hollow body (11). Said outer surface is impermeable to liquids, but permeable to gases. Therefore, said outer surface is able to provide a liquid impermeable barrier, while allowing gas venting. Said outer surface may be liquid impermeable up to pressures differences of 1 bar between the inside and the outside of said hollow body, preferably up to pressures differences of 500 mbar. Said outer surface may be a planar surface, at least when viewed macroscopically. Said outer surface may also comprise a network of microchannels which is permeable to gases, but not to liquids, as described in EP-A-593 840. Said outer surface may be corrugated macroscopically, like a zigzagged surface, in which case said outer surface is defined by several planes of different inclination with respect to the horizontal direction, connected to each other.

Preferably, said outer surface is made of a membrane (22a). In the following, the wording "outer surface" is equivalent with "membrane". Preferably, said membrane is any material capable of being formed into a thin layer which may be used to cover said orifice (21). Said membrane must be permeable to gas flow, also in response to small pressure differences. Preferably, said membrane should allow gas flow with pressure differences as low as 50 mbar, more preferably as low as 5 mbar. The thickness of said membrane is a matter of choice, but preferably would be in the region of 0.2 mm to 2 mm. Said membrane can comprise essentially any material which may be formed into thin layers such as plastics, paper or metal having micropores. Preferred materials for said membrane include microporous plastic films. The size of the micropores of said membrane should be such so as to allow the passage of gases at low pressure differences and at the same time to provide a high level of liquid impermeability. Preferably, the micropores will be in the range of 0.1 µm to 5 µm, more preferably between 0.2 µm to 1 µm. Preferably, said membrane has a rounded shape. But other shapes, such as rectangular, triangular or else, may be also foreseen to adapt it in a package or cap and/or improve the aesthetics of the package or cap itself.

Preferred microporous plastic films for this application are:
- non-woven plastic films, especially the non-woven spun bounded polyethylene film material sold under the trade name TYVEK by the Du Pont Company, of which TYVEK, Style 10, which is fluorocarbon treated to achieve high fluid impermeability;
- an acrylic copolymer cast on a non-woven support (nylon or PET) with a fluoro-monomer post-treatment hydrophobicity, sold under the trade name, VERSAPOR, by the Gelman Sciences Company, 600, South Wagner Road, Ann Arbor, MI 48106, US.

The microporous film material of said membrane (22a) may be treated to reduce its surface energy and therefore to improve the leak tightness of said film material. The lowering of the surface energy of said film material is particularly necessary to improve leak tightness when said package (10) contains products comprising surfactant components. Preferably in this case, the specific surface energy of said film material should be lower than that of the surfactant-containing product to achieve a substantially complete impermeability to the product contents.

Fluorocarbon treatment, which involves fixation of a fluorocarbon material, on a micro scale, to the surface of the film material is a specific example of a treatment which provides such reduced surface energy. Indeed, the fluorination treatment reduces the susceptibility of the microporous film material of said membrane to wetting by the liquid product contents. However, when used to treat said microporous film material of said membrane according to the present invention, this fluorocarbon treatment should not compromise the gas permeability of said membrane. For example, a possible fluorocarbon material for use in the fluorocarbon treatment according to the present invention is sold under the trade name SCOTCHBAN, by the 3M Company.

Said membrane (22a) may be applied and located inside said hollow body (11) between the content and said orifice (21) in any way maintaining its liquid-impermeability and gas-permeability according to the present invention. The means of application may therefore include the use of adhesives, or heat-sealing of said membrane onto the area around said orifice or mechanical means such as clamping or hot-stamping, or insertion of said membrane during molding of said package. As said before, the application means employed should not significantly compromise the venting ability of the membrane. For this reason, it is preferred that any adhesive used is also permeable to gases, or does not fill up the pores of the membrane.

The membrane (22a) may be also fitted in a housing. Housings whose dimensions are particularly compatible for use in a package or a cap according to the present invention are commercially available from GVS, Via Roma 50, 40069, Zola Predosa (BO), Italy. In a highly preferred embodiment, the manufacture of said housing and the fitting of said membrane (22) in said housing can be achieved by an "insert molding operation", where:
- a sheet of membrane is fed into an apparatus; the sheet of membrane is advantageously fed from a roll of membrane material;
- in said apparatus, at least one membrane is cut from said sheet and is placed into a mold wherein said housing will be formed;
- then, the housing is molded substantially around said membrane in a manner which secures said membrane in said housing. As "substantially around" it is meant herein that once completed, this step should generate a housing with its fitted membrane, where both surfaces of the membrane are accessible to air, but said membrane is tightly maintained in the housing.

Housings may also be manufactured by heat sealing, ultrasonic sealing or gluing said membrane (22a) into said housing. Furthermore, housings may be manufactured by mechanically holding the membrane between two separate pieces whereby said pieces are clipped together.

As explained hereinabove, the venting performance of said venting means (20) may be substantially reduced when the contained liquid product contacts said outer surface (22) or said membrane (22a). As explained above, said outer surface or membrane is the most exposed part of said venting means towards the contained product. The contacting between said product and said membrane inside a package may mainly occur through splashes during shipment and transportation with agitation of said package. As used herein "splashing" means a non-continuous and brief contact of a liquid substance upon a surface when said liquid is agitated within the package. The splashing of the contained liquid product occurs mainly during shipment and transportation, when the risk of agitation of said package is higher.

We found that these membranes may lose their gas-permeability when the contained liquid product contacts said membrane (22a). Indeed, we found that liquid product or part of said product may not sufficiently drain away from said membrane. In this manner, said membrane may be covered by the product, i.e. its venting performance is reduced for any part of said membrane covered by the product which has not drained away. Consequently, the venting capacity of the package is reduced or effectively lost.

This is particularly the case for liquid products which are viscous, or which have some affinity for the membrane. We found that products having viscosities of at least 5 cps when measured using a Brookfield viscosity meter at 60 rpm, spindle 3 and 20° Celsius demonstrate poor drainage away from said membrane. Other examples are liquids exhibiting shear thinning, non-newtonian flow behaviour or liquids having a low surface energy (< 30 dyne/cm²). For example, liquids comprising surfactants exhibit typically a non-newtonian flow behaviour. As used herein, a "shear thinning" product is a product which presents a high viscosity when the shear rate is low, and vice versa its viscosity is low when the shear rate is high. A shear thinning (non-newtonian) product exhibits poor drainage away from said membrane. This is because, due to the product flow characteristics observed during drainage, the shear rate of product directly adjacent to the membrane is low. Consequently, the final layer of product adjacent to the membrane exhibits an intrinsically high viscosity. Therefore, the drainage of the final layer of product away from the membrane is impeded.

The contacting between said contained liquid product and said outer surface (22) or membrane (22a) occurs mainly during shipment and transportation of the package. Indeed, said liquid product splashes onto said membrane within said package when said package is agitated. We found that the amount of splashes normally occurring during shipment and transportation are sufficient to completely interrupt the venting capacity of said package. Another means by which product may contact with the membrane is during an upside down storage of the package. We further found that other venting systems, like valves for example, may also suffer from a similar disadvantage. Consequently, the present invention provides a package for a liquid product, or a cap for such a package which enables or compels the drainage of said splashed product away from said membrane. We found that the package or cap according to the present invention enables sufficient venting of said product to prevent significant package deformation and without any substantial loss of venting capacity.

A possible way to remove the splashed product from the membrane is to scrape the surface of the membrane splashed by said product. We found that the venting capacity of said membrane recovered sufficiently to prevent significant bottle deformation once said splashed product was removed from the surface of said membrane. The scraping of said surface may be achieved with a device having the form of a shovel, for example. Although this solution solves the problem of the present invention, it has two major disadvantages. Firstly, the scraping action has to be carried out either manually by the user, which is inappropriate, or by a mechanical moving device within the package, which may be complex and expensive. Secondly, the action of scraping said splashed product from said membrane may damage said membrane. Indeed, especially the impermeability of said membrane to liquids may be easily lost through scraping. Therefore, the present invention further provides a package or a cap in which said splashed product is enabled or compelled to drain away from said membrane automatically without any scraping of said membrane.

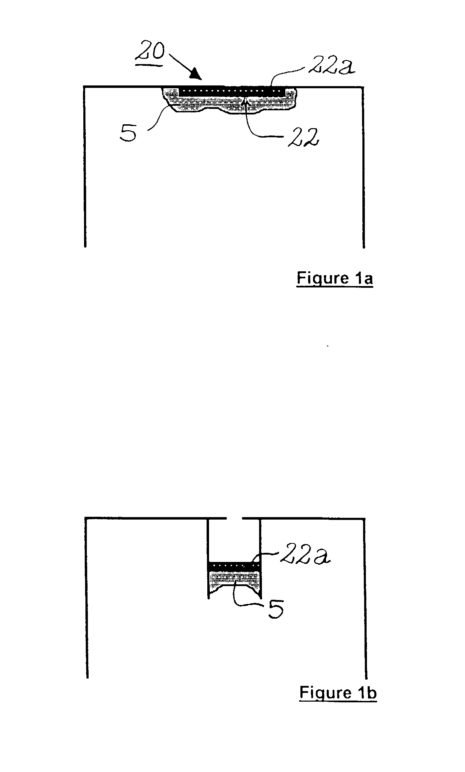

Figure 1a shows a horizontal outer surface (22) or horizontal membrane (22a) which has been splashed by a viscous product. In this case, the product (5) has failed to drain away from said membrane. This is because the viscosity of the product is sufficiently high that, firstly, gravity is insufficient in itself to compel said product to drain away from said membrane, and secondly, as another possible reason, the hydrophobic nature of the membrane is insufficient to repel the product away from said membrane. The drainage of said splashed product (5) away from said membrane can be further impeded when the membrane is completely surrounded by a region of the package, as represented in Figure 1b. This region may exist as part of a housing of said membrane, as part of the package or as part of said cap. This region can prevent splashed product from draining away from the membrane. This is because the capillary forces exerted by said region on said splashed product, the viscosity of the product and the surface tension of said product are sufficient to retain the splashed product within said region around said membrane.

A means (30) which is able to solve the case illustrated in Figure 1a is to position said outer surface (22) or said membrane (22a) substantially vertical or inclined in said package or cap (10), as shown in Figure 2a (cross sectional view side and front view). Preferably, said venting means, comprising said membrane and said orifice (21), is located on the side wall (18) of said package or cap. Accordingly, said venting means is positioned on the side wall (18) on the upper portion of said package or cap such that said venting means is at least above the level of the contained liquid product. This substantially vertical or inclined configuration of said venting means improves product drainage from said membrane enabling said venting means to vent. Indeed, the force of gravity is able to act onto said splashed product by forcing said product to drain towards the lowest edge (20a) of said venting means away from said outer surface or membrane. As defined hereinafter, the "attractive forces" are those forces which act onto said splashed product and retain said product onto said outer surface. We found that the component of the attractive forces parallel to said outer surface is smaller than the corresponding component of the force of gravity. Therefore, said splashed product is forced towards said lowest edge (20a) away form said outer surface.

We found that at least said outer surface (22) or said membrane (22a) should be positioned substantially vertical or inclined to improve drainage. Examples are given in Figure 2c and Figure 2d. The orifice (21) is on the top wall (17). Said outer surface or membrane covers said orifice in such a manner to expose always substantially vertical or inclined surfaces towards the content of said package or cap when said package stands in its upright position. Inclined membranes (22a) may be also achieved on housings (6), as illustrated in Figure 2e and Figure 2f. In these cases, said membrane may be fitted either to the side wall (Fig. 2e, 18a) or to the end of said housing (Fig. 2f), these being the most exposed positions to said contained product. The same fittings methods are applicable as those described above. Preferably, said housing (6) is engaged with the wall (19) to said package or cap. The housing itself may be placed either to said top wall (17) or to said side wall (18) of said package or cap (10). Another way to achieve inclined membranes according to the present invention may be obtained by a venting means which is part of a cap, and said cap (10a) is engaged to an inclined neck (10b) of a container or bottle (10c), as depicted in Figure 2g.

We found that the drainage from a vertical or inclined venting means or membrane may be further improved by a means which increases the surface energy difference between said outer surface (22) (or said membrane (22a)), and the region of the package surrounding said outer surface (or said membrane). The membrane has a lower surface energy than the product to ensure leak-tightness of said product. As a consequence, said product is repelled away from said membrane (22a). Therefore, drainage of product away from said membrane can be improved by ensuring that the region of the package surrounding said outer surface of said membrane has a higher surface energy than that of the product. This is because, said product will preferentially wet the surfaces of regions in the package which exhibit a higher surface energy than that of the product. Conversely, said product is repelled away from regions of the package which possess a lower surface energy than the product. Therefore, by ensuring that the region of the package surrounding said outer surface (22) has a higher surface energy than said product, drainage of said product is improved.

Increasing the surface energy of the region surrounding said outer surface (22) or said membrane (22a) can be achieved in a number of ways. Firstly, the membrane may be surrounded by a peripheral wall (32), as shown in Figure 2a, said peripheral wall (32) being made of a material which has more affinity with said product than said membrane (22). For example, the surface energy may be increased by having said peripheral surface made of a material which is more hydrophilic than said outer surface or membrane. Such peripheral walls may be inserted into the package as a separate plastic piece, or may be applied as part of a surface coating, or may be co-injected during package manufacture into the region surrounding said membrane. Secondly, the package or cap for said package which contains the membrane may itself be made from a material which has more affinity with said product than said membrane. Thirdly, the region in a housing which surrounds said membrane may also be made of such a material, as, for example, shown in Figure 2d. Preferably, said region which surrounds said membrane extends from and is connected to at least said membrane (22a). More preferably, said peripheral surface is a parallel extension of said outer surface or said membrane.

Another means which improves the drainage of product away from said outer surface (22) or of said membrane (22a) as shown in Figure 1a or when said membrane is within a housing, as depicted in Figure 1b, is a reduction of the capillary attractive force between said product and the region surrounding said membrane, and therefore enabling the force of gravity to drain said product away from said membrane. A further means, which improves the drainage of said splashed product away from said membrane, is an increase of the shear rate acting on said product. Consequently, this means allows to reduce the viscosity of products having non-newtonian flow behaviour, and therefore increase the flow capability of said product. Both these means can be applied to horizontal or non-horizontal outer surfaces or membranes of said venting means with and without said peripheral surface.

A possible way to solve the case shown in Figure 1a, which also solves the case shown in Figure 1b, is to position a draining means (35) close to the venting region or close to said membrane (22a). Preferably, said draining means is located partially around said membrane and/or peripheral surface (32). Preferably, said draining means is located at the lowest edge of said membrane and/or peripheral surface when said package or cap is in its upright position. The draining means (35) according to the present invention has to satisfy certain constraints so that it does not interfere with the other functions of a package. Firstly, said draining means should not interfere with the air flow from the product through the venting system, i.e. it should not in itself interrupt the venting of the package. Secondly, said draining means should not interfere with the dispensing of the product from the interior of the package.

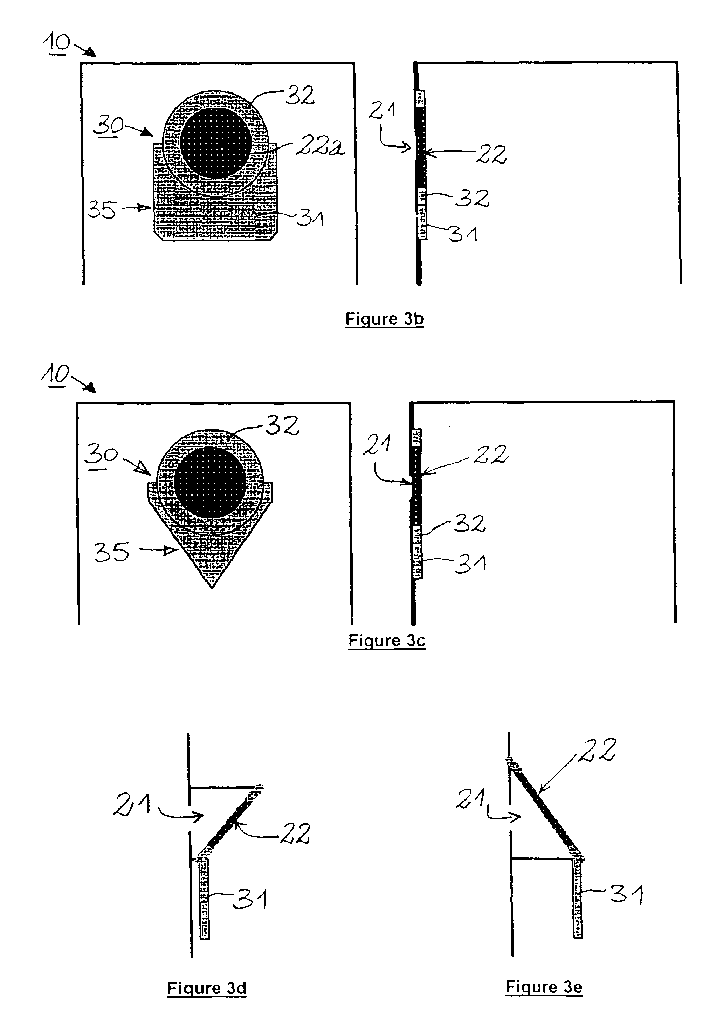

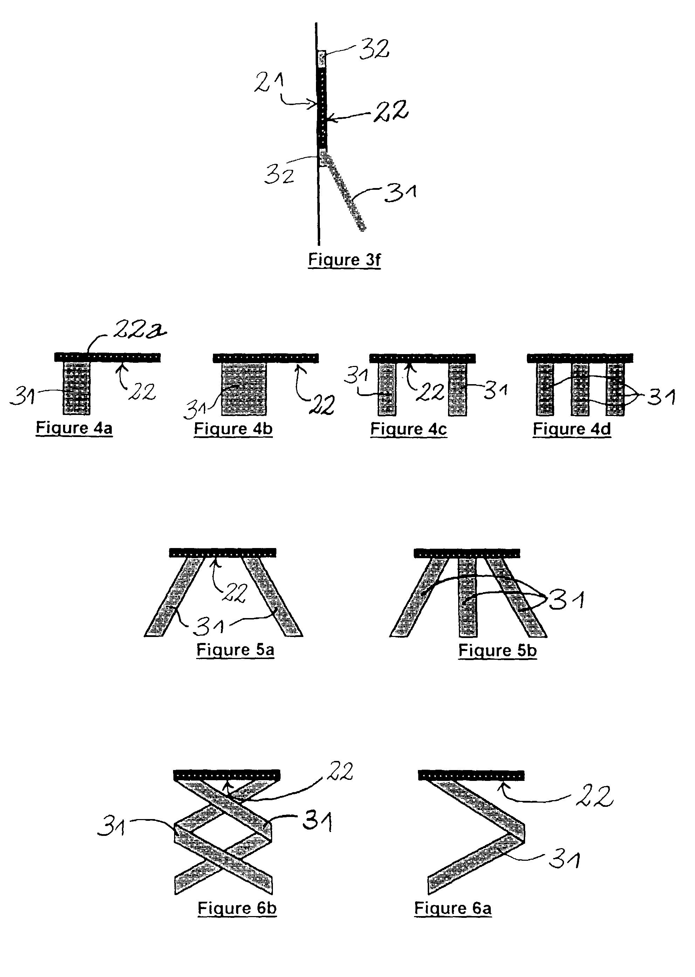

Said draining means (35) will be further illustrated by the following non-limiting examples with the help of Figures 3. Figure 3a illustrates a cross sectional side view and a front view of a venting means positioned substantially vertically on said package (partially shown) or cap (10), preferably on said side wall (18). Said venting means comprises an orifice (21) and the outer surface (22) or membrane (22a). Said draining means comprises a wick (31) extending from said venting means down towards the inside of said package or cap. Preferably, said wick is attached to the lowest edge of said outer surface or membrane and/or said peripheral surface (32). Alternatively, said wick may be part or attached to said package or cap near the location of said membrane, or part or attached to the spout of said package or cap whereby said wick is in close proximity to said membrane and/or peripheral surface at least at its lowest edge. Preferably, said wick is located within one drop diameter to said membrane and/or peripheral surface, more preferably between 1 mm and 6 mm to said membrane and/or peripheral surface.

The contact of said wick (31) with said membrane (22) and/or with said peripheral surface (32), is critical for the present invention. Indeed, without being limited by any theory, we believe firstly that said wick is able to reduce the capillary force, since said wick forms a geometry which is asymmetric at the surface of the venting means or membrane. This asymmetry at the surface of said membrane has the effect that the liquid is compelled to drain away from the surface of said membrane without being blocked by the capillary force.

Secondly, the geometry of said wick (31) causes the product to drain away from said membrane by increasing the shear rate at least locally in those position(s) where said wick contacts said membrane. This means that a flowing product which exhibits shear thinning, non-newtonian flow behaviour has a decreased viscosity. Therefore, said wick further improves the drainage of this type of product away from said venting means.

To further improve the drainage, we believe that said wick (31) is able to attract said product from around said membrane, if the wick has a higher surface energy than either said product or said outer surface or membrane. This can be achieved with a wick made of a material which is more hydrophilic than said membrane. Indeed, the hydrophilicity of said wick should be such to further attract product away from said membrane by an increased surface energy balance, as explained above for said peripheral surface (32). Consequently, because of this attraction, said drained liquid is able to further flow away from said membrane. At the same time, this allows other liquid, still on said membrane, to flow over said membrane onto said wick. The liquid coming together on said wick then runs down said wick and collects at point 35a where it eventually reaches such a weight so as to drop back inside said package.

The form of the surface of said wick (31) may be various, as shown in Figures 3a to 3e. The surface may be of triangular, rectangular or rounded form. For example, in Figure 3a said wick is a slender rectangular. As depicted in Figures 3b and 3c, said wick can be also shaped as a shovel. Specifically, said shovel may have a triangular or rectangular shape and optionally a rounded periphery. The person skilled in the art may choose the appropriate form and dimension of said draining means to adapt it in a package or cap and/or to improve the aesthetics of the package or cap.

Another possibility is to incline said outer surface (22) and/or membrane (22a) on said package or cap (10) with respect to the vertical direction, as shown in Figures 3d and 3e. Preferably, the inclination angle α of said membrane with respect to the vertical direction is preferably between 10°deg and 90°deg, more preferably between 30°deg and 70°deg. As depicted in Figure 3f, said wick (31) may also be inclined with respect to the vertical direction. Said wick may be inclined also with respect to said outer surface. Preferably, the inclination angle β of said wick with respect to said vertical direction is preferably between 10°deg and 90°deg, more preferably between 30°deg and 70°deg. Most preferably, said wick is inclined away from said membrane, such that the smallest angle δ between said membrane and said wick is greater than 90°deg. Indeed, we found that this geometry between said membrane and said wick further compels the drainage away of product from said membrane.

We further found that said draining means (35) substantially improves the drainage away from a non-inclined or horizontal outer surface (22) or membrane (22a), i.e. from a venting means being positioned horizontally on the top wall (17) of said package or cap, as shown in Figure 4a. Said draining means may comprise again a wick (31) which starts from the lowest edge of said membrane and extends from said venting means down towards the inside of said package or cap. Said draining means will be further illustrated by the following non-limiting examples with the help of the Figures 4 to 7. The same forms of said draining means as described in Figures 3 are directly applicable to the horizontal venting means.

Furthermore, several wicks (31) in different configurations may be placed around the perimeter of said outer surface (22) or membrane (22a), as depicted in Figures 4a to 4d. For example, wicks in the form of a rectangular or of an arc may be disposed singularly or in groups of two or more. The single wick of Figures 4a and 4b may partially enclose the perimeter (23) of membrane. A wick enclosing completely the whole perimeter of said venting means may be also envisaged. Two or more wicks may divide in a regular manner said perimeter, as shown in Figures 4c and 4d.

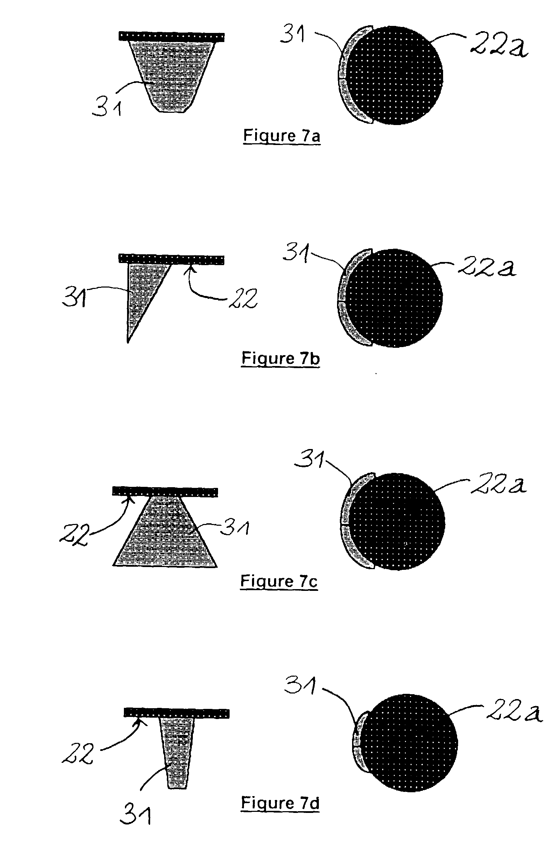

Inclination of said wicks (31) with respect to the vertical direction is also possible, as illustrated in Figures 5a and 5b. Another possibility is to have wicks in the form of slides, as depicted in Figures 6a and 6b. Figure 6a represents a single wick in the form of a slide turning down towards the inside of said package or cap, like a spiral. Figure 6b shows the possibility of having more than one wick in the form of slides. Each wick turns down opposing each other. In Figures 7a to 7d are other possible forms of said wick at least partially surrounding said membrane, as shown in the corresponding bottom views. Specifically, forms like a shovel (Fig. 7a), a triangle (Fig. 7b) or polygonal (Fig. 7c and 7d). The person skilled in the art may choose the appropriate form and dimension of said draining means to adapt it in a package or cap and/or to improve the aesthetics of the package or cap itself.

Preferably, said draining means is made of plastic, metal, paper, or combinations of these materials as layers, laminates or co-extrudates. The materials may be also recycled. Preferred materials for said draining means include all materials having a higher hydrophilicity with respect to said membrane (22) again to increase the surface energy balance as explained above. Such materials are, for example, plastics such as polyethylene (high or low density), polyvinyl chloride, polyester, polyethylene terephthalate (=PET), extrudable PET, polypropylene, polycarbonate and nylon. These plastics may used individually or be combined as co-extrudates, layers or laminates. Another possibility is to render hydrophobic materials more hydrophilic using either additives to the bulk material or appropriate surface coatings.

Draining means (35) may be inserted into packages using a variety of means. They may be inserted into the package during the molding of the package or cap (10). Also, they may be manufactured as part of a housing (6) containing said membrane (22a). Furthermore, they can be inserted as a separate item into the package after said package has been manufactured. The means of attaching said separate piece can be achieved using adhesive or a welding operation. Also, this separate piece can be achieved using a mechanical holding device, i.e. the draining means may be manufactured as part of a piece which acts as a mechanical holding device for the membrane. For example, said draining means may be incorporated onto a clip which holds the membrane in place.

Accordingly, the present invention enables the venting by said venting means (20) comprised in a package for a liquid product, or a cap for such a package, whereby it enables or compels said product to drain away from said venting means. The present invention is especially valuable for products having a certain viscosity or exhibiting shear thinning, non-newtonian flow behaviour or for products having a low surface energy (< 30 dyne/cm²) as described above. Said invention is able to reduce the capillary attractive force, and therefore enabling the force of gravity to act onto said product. Said invention provides also a means to increase at least locally the shear rate acting on said product during drainage of said product from said outer surface (22) or membrane (22a). Said invention further provides a means to increase the surface energy balance between said outer surface or membrane and the region surrounding said outer surface or said membrane. All these features may be combined with each other in a suitable manner within the scope of the appended claims to improve the drainage of said product away from said outer surface of said membrane.

## Claims

1. A package (10) containing a liquid product, said product having a viscosity of at least 5 cps measured when using a Brookfield viscosity meter at 60 rpm, spindle 3 and 20° Celsius, said package enabling the venting of said product by a venting means (20), said venting means allowing passage of gases between the interior and the exterior of said package when the pressure inside said package differs from the ambient pressure, said venting means being permeable to gases but impermeable to said product, and said venting means comprising an outer surface, said package comprising a means (30) which enables said product to drain away from said outer surface of said venting means, once said product has come in contact with said outer surface of said venting means.

2. A package (10) containing a liquid product according to claim 1, wherein said package is closed by a cap, said venting means being positioned onto said cap (10).

3. A package according to claims 1 and 2 characterized in that said draining means (35) extends from and is connected to at least said outer surface of said venting means, and said draining means extends in an inclined direction with respect to the horizontal direction.

4. A package (10) for a liquid product, or a cap (10) for such a package, said product having a viscosity of at least 5 cps measured when using a Brookfield viscosity meter at 60 rpm, spindle 3 and 20° Celsius, said package or cap enabling the venting of said product by a venting means (20), said venting means allowing passage of gases between the interior and the exterior of said package when the pressure inside said package differs from the ambient pressure, said venting means being permeable to gases but impermeable to said product, and said venting means comprising an outer surface, said package or cap comprising a means (30) which enables said product to drain away from said outer surface of said venting means, once said product has come in contact with said outer surface of said venting means, said means (30) comprising a draining means (35) that is positioned close to a venting region or membrane (22a) of the venting means, and said draining means being made out of a material with higher surface energy compared to the surface energy of the outer surface of the venting means or the surface energy of the membrane (22a), characterized in that said means (30) comprises the positioning of said venting means (20) and/or an outer surface (22) of said venting means in a plane inclined with respect to the horizontal direction.

5. A package or cap according to any of the preceding claims characterized in that said means (30) comprises a reduction of the capillary attractive force between said product and the region surrounding said venting means (20), and therefore enabling the force of gravity, acting onto said product, to be sufficient to cause said product to drain away from said outer surface (22) of said venting means, said reduction of the capillary attractive force being achieved by said training means (35).

6. A package or cap according to any of the preceding claims characterized in that said means (30) comprises an at least local increase of the shear rate acting on said product during drainage of said product from said outer surface (22) of said venting means (20), and therefore reduces the viscosity of products having shear thinning, non-newtonian flow properties, said at least local increase of the shear rate being achieved by said draining means (35).

7. A package or cap according to any of the preceding claims characterized in that said means (30) comprises an increase of the surface energy balance between said outer surface (22) of said venting means (20) and the region surrounding said outer surface of said venting means to enhance the drainage of said product away from said outer surface of said venting means.

8. A package or cap according to claim 7 characterized in that said means (30) comprises a material which has more affinity with said product than said outer surface (22) of said venting means.

9. A package or a cap according to any of claims 7 and 8 characterized in that said venting means (20) comprises a peripheral surface (32), said peripheral surface extending from and connected to at least said outer surface (22) of said venting means, said peripheral surface being a parallel extension of said outer surface of said venting means, and said peripheral surface being more hydrophilic than said outer surface of said venting means.

10. A package or cap according to claim 4 characterized in that said means (30) comprises a substantially vertical positioning of said venting means (20) and/or of said outer surface (22) of said venting means.

11. A package or a cap according to any of claims 4 to 10 characterized in that the inclination angle α of said venting means and/or said outer surface of said venting means is between 10°deg and 90°deg.

12. A package or a cap (10) according to any of the preceding claims characterized in that said draining means (35) is inclined in a substantially vertical manner with respect to the outer surface of said venting means.

13. A package or a cap according to any of the preceding claims characterized in that the inclination angle β of said draining means (35) with respect to the horizontal direction is between 10°deg and 90°deg.

14. A package or a cap according to any of the preceding claims characterized in that said draining means (35) is made of a material which is more hydrophilic than said outer surface (22) of said venting means (20).

15. A package or a cap according to any of the preceding claims characterized in that said draining means (35) further comprises a wick (31) extending from said venting means down towards the inside of said package or cap.

16. A package or a cap according to any of the preceding claims characterized in that said venting means (20) comprises an orifice (21) connecting the interior with the exterior of said package, and a membrane (23) covering said orifice or a portion thereof which permits the passage of gases, but prevents the passage of liquid products.

17. A package or a cap according to any of the preceding claims characterized in that said product has viscosity of between 200 cps and 5000 cps measured when using a Brookfield viscosity meter at 60 rpm, spindle 3 and 20° Celsius.

18. A package or a cap according to any of the preceding claims characterized in that said product comprises bleach.

19. A package or a cap according to claim 18 characterized in that said product comprises peroxygen bleach.

## Patentansprüche

1. Verpackung (10), enthaltend ein flüssiges Produkt, wobei das Produkt eine Viskosität von mindestens 5 cps aufweist, gemessen unter Verwendung eines Brookfield-Viskosimeters mit 60 U/min, einer 3er Spindel und bei 20°C, wobei die Verpackung das Ventilieren bzw. Entgasen des Produkts durch eine Ventiliereinrichtung (20) ermöglicht, wobei die Ventiliereinrichtung das Hindurchströmen von Gasen zwischen dem Inneren und dem Äußeren der Verpackung zuläßt, wenn der Druck innerhalb der Verpackung von dem Umgebungsdruck verschieden ist, wobei die Ventiliereinrichtung für Gase durchlässig ist, hingegen für das Produkt undurchlässig ist, und wobei die Ventiliereinrichtung eine Außenfläche umfaßt, wobei die Verpackung eine Einrichtung (30) umfaßt, die ein Ableiten des Produkts von der Außenfläche der Ventiliereinrichtung erlaubt, nachdem das Produkt mit der Außenfläche der Ventiliereinrichtung in Kontakt gekommen ist.

2. Verpackung (10), enthaltend ein flüssiges Produkt gemäß Anspruch 1, wobei die Verpakkung durch einen Kappenverschluß verschlossen ist, wobei die Ventiliereinrichtung auf dem Kappenverschluß angebracht ist (10).

3. Verpackung gemäß Anspruch 1 und 2, dadurch gekennzeichnet, daß die Ableitungseinrichtung (35) sich von der Außenfläche der Ventiliereinrichtung erstreckt und zumindest mit der Außenfläche verbunden ist, und die Ableitungseinrichtung in geneigter Richtung bezüglich der horizontalen Richtung verläuft.

4. Verpackung (10) für ein flüssiges Produkt oder ein Kappenverschluß (10) für eine solche Verpackung, wobei das Produkt eine Viskosität von mindestens 5 cps aufweist, gemessen unter Verwendung eines Brookfield-Viskosimeters mit 60 U/min, einer 3er Spindel und bei 20°C, wobei die Verpackung oder der Kappenverschluß das Ventilieren des Produkts durch eine Ventiliereinrichtung (20) ermöglichen, wobei die Ventiliereinrichtung das Hindurchströmen von Gasen zwischen dem Inneren und dem Äußeren der Verpackung zuläßt, wenn der Druck innerhalb der Verpackung von dem Umgebungsdruck verschieden ist, wobei die Ventiliereinrichtung für Gase durchlässig ist, hingegen für das Produkt undurchlässig ist und wobei die Ventiliereinrichtung eine Außenfläche umfaßt, wobei die Verpackung oder der Kappenverschluß eine Einrichtung (30) umfaßt, die ein Ableiten des Produkts von der Außenfläche der Ventiliereinrichtung erlaubt, nachdem das Produkt mit der Außenfläche der Ventiliereinrichtung in Kontakt gekommen ist, wobei die Einrichtung (30) eine Ableitungseinrichtung (35) umfaßt, welche nahe einer Ventilierregion oder Membran (22a) der Ventiliereinrichtung positioniert ist, und die Ableitungseinrichtung aus einem Material mit einer höheren Oberflächenenergie im Vergleich zu der Oberflächenenergie der Außenfläche der Ventiliereinrichtung oder der Oberflächenenergie der Membran (22a) besteht, dadurch gekennzeichnet, daß die Einrichtung (30) die Positionierung der Ventiliereinrichtung (20) und/oder einer Außenfläche (22) der Ventiliereinrichtung in einer Ebene, die bezüglich der horizontalen Richtung geneigt ist, umfaßt.

5. Verpackung oder Kappenverschluß gemäß mindestens einem der vorhergehenden Ansprüche, dadurch gekennzeichnet, daß die Einrichtung (30) die Verringerung der Kapillaranziehungskraft zwischen dem Produkt und der die Ventiliereinrichtung (20) umgebenden Region umfaßt und deshalb ermöglicht, daß die auf das Produkt einwirkende Schwerkraft ausreicht, um für die Ableitung des Produkts von der Außenfläche (22) der Ventiliereinrichtung zu sorgen, wobei die Verringerung der Kapillaranziehungskraft durch die Ableitungseinrichtung (35) erreicht wird.

6. Verpackung oder Kappenverschluß gemäß mindestens einem der vorhergehenden Ansprüche, dadurch gekennzeichnet, daß die Einrichtung (30) zumindest eine lokale Erhöhung der Scherrate umfaßt, die auf das Produkt während der Ableitung des Produkts von der Außenfläche (22) der Ventiliereinrichtung (20) einwirkt und daher die Viskosität von Produkten mit Scherentzähungs- und Nicht-Newtonschen Fließeigenschaften verringert, wobei zumindest eine lokale Erhöhung der Scherrate durch die Ableitungseinrichtung (35) erzielt wird.

7. Verpackung oder Kappenverschluß gemäß mindestens einem der vorhergehenden Ansprüche, dadurch gekennzeichnet, daß die Einrichtung (30) die Zunahme des Oberflächenenergiegleichgewichts zwischen der Außenfläche (22) der Ventiliereinrichtung (20) und der die Außenfläche umgebenden Region der Ventiliereinrichtung umfaßt, um die Ableitung des Produkts von der Außenfläche der Ventiliereinrichtung zu verbessern.

8. Verpackung oder Kappenverschluß gemäß Anspruch 7, dadurch gekennzeichnet, daß die Einrichtung (30) ein Material umfaßt, welches mehr Affinität mit dem Produkt als mit der Außenfläche (22) der Ventiliereinrichtung aufweist.

9. Verpackung oder Kappenverschluß gemäß mindestens einem der Ansprüche 7 und 8, dadurch gekennzeichnet, daß die Ventiliereinrichtung (20) eine periphere Oberfläche (32) umfaßt, wobei die periphere Oberfläche sich von der Außenfläche (22) der Ventiliereinrichtung erstreckt und zumindest mit der Außenfläche (22) verbunden ist, wobei die periphere Oberfläche eine parallele Verlängerung der Außenfläche der Ventiliereinrichtung ist und die periphere Oberfläche hydrophiler ist als die Außenfläche der Ventiliereinrichtung.

10. Verpackung oder Kappenverschluß gemäß Anspruch 4, dadurch gekennzeichnet, daß die Einrichtung (30) eine praktisch vertikale Positionierung der Ventiliereinrichtung (20) und/oder der Außenfläche (22) der Ventiliereinrichtung umfaßt.

11. Verpackung oder Kappenverschluß gemäß mindestens einem der Ansprüche 4 bis 10, dadurch gekennzeichnet, daß der Neigungswinkel α der Ventiliereinrichtung und/oder der Außenfläche der Ventiliereinrichtung zwischen 10 Grad und 90 Grad beträgt.

12. Verpackung oder Kappenverschluß (10) gemäß mindestens einem der vorhergehenden Ansprüche, dadurch gekennzeichnet, daß die Ableitungseinrichtung (35) praktisch vertikal bezüglich der Außenfläche der Ventiliereinrichtung geneigt ist.

13. Verpackung oder Kappenverschluß gemäß mindestens einem der vorhergehenden Ansprüche, dadurch gekennzeichnet, daß der Neigungswinkel β der Ableitungseinrichtung (35) bezüglich der horizontalen Richtung zwischen 10 Grad und 90 Grad beträgt.

14. Verpackung oder Kappenverschluß gemäß mindestens einem der vorhergehenden Ansprüche, dadurch gekennzeichnet, daß die Ableitungseinrichtung (35) aus einem Material besteht, welches hydrophiler als die Außenfläche (22) der Ventiliereinrichtung (20) ist.

15. Verpackung oder Kappenverschluß gemäß mindestens einem der vorhergehenden Ansprüche, dadurch gekennzeichnet, daß die Ableitungseinrichtung (35) weiterhin einen Docht (31) umfaßt, welcher sich von der Ventiliereinrichtung aus hinab zu der Innenseite der Verpackung oder des Kappenverschlusses erstreckt.

16. Verpackung oder Kappenverschluß gemäß mindestens einem der vorhergehenden Ansprüche, dadurch gekennzeichnet, daß die Ventiliereinrichtung (20) eine Austrittsöffnung (21) umfaßt, welche das Innere mit dem Äußeren der Verpackung verbindet, und eine Membran (23), welche die Austrittsöffnung oder einen Teil davon bedeckt, welche den Durchlaß von Gasen ermöglicht, hingegen den Durchlaß von flüssigen Produkten verhindert.

17. Verpackung oder Kappenverschluß gemäß mindestens einem der vorhergehenden Ansprüche, dadurch gekennzeichnet, daß das Produkt eine Viskosität zwischen 200 cps und 5000 cps aufweist, gemessen unter Verwendung eines Brookfield-Viskosimeters mit 60 U/min, einer 3er Spindel und bei 20°C.

18. Verpackung oder Kappenverschluß gemäß mindestens einem der vorhergehenden Ansprüche, dadurch gekennzeichnet, daß das Produkt Bleichmittel beinhaltet.

19. Verpackung oder Kappenverschluß gemäß Anspruch 18, dadurch gekennzeichnet, daß das Produkt Peroxidbleichmittel beinhaltet.

## Revendications

1. Emballage (10) contenant un produit liquide, ledit produit ayant une viscosité d'au moins 5 cps telle que mesurée en utilisant un viscosimètre de Brookfield à 60 tpm, broche 3 et à 20°Celsius, ledit emballage permettant la purge dudit produit via des moyens de purge (20), lesdits moyens de purge permettant le passage de gaz entre l'intérieur et l'extérieur dudit emballage lorsque la pression à l'intérieur dudit emballage diffère de la pression ambiante, lesdits moyens de purge étant perméables aux gaz, mais imperméables audit produit, et lesdits moyens de purge comportant une surface extérieure, ledit emballage comportant des moyens (30) qui permettent audit produit de s'écouler loin de ladite surface extérieure desdits moyens de purge, lorsque ledit produit est venu en contact avec ladite surface extérieure desdits moyens de purge.

2. Emballage (10) contenant un produit liquide selon la revendication 1, dans lequel ledit emballage est fermé par un bouchon, lesdits moyens de purge étant positionnés sur ledit bouchon (10).

3. Emballage selon les revendications 1 et 2, caractérisé en ce que lesdits moyens de drainage (35) s'étendent à partir d'au moins ladite surface extérieure desdits moyens de purge et lui sont reliés, et lesdits moyens de drainage s'étendent dans une direction inclinée par rapport à la direction horizontale.

4. Emballage (10) pour un produit liquide, ou bouchon (10) pour un tel emballage, ledit produit ayant une viscosité d'au moins 5 cps telle que mesurée en utilisant un viscosimètre de Brookfield à 60 tpm, broche 3 et à 20°Celsius, ledit emballage ou bouchon permettant la purge dudit produit via des moyens de purge (20), lesdits moyens de purge permettant le passage de gaz entre l'intérieur et l'extérieur dudit emballage lorsque la pression à l'intérieur dudit emballage diffère de la pression ambiante, lesdits moyens de purge étant perméables aux gaz, mais imperméables audit produit, et lesdits moyens de purge comportant une surface extérieure, ledit emballage ou bouchon comportant des moyens (30) qui permettent audit produit de s'écouler loin de ladite surface extérieure desdits moyens de purge, lorsque ledit produit est venu en contact avec ladite surface extérieure desdits moyens de purge, lesdits moyens (30) comportant des moyens de drainage (35) qui sont positionnés à proximité d'une région ou membrane de purge (22a) des moyens de purge, et lesdits moyens de drainage étant constitués d'un matériau qui possède une énergie superficielle plus élevée par comparaison avec l'énergie superficielle de la surface extérieure des moyens de purge ou l'énergie superficielle de la membrane (22a), caractérisé en ce que lesdits moyens (30) comprennent le positionnement desdits moyens de purge (20) et/ou d'une surface extérieure (22) desdits moyens de purge dans un plan incliné par rapport à la direction horizontale.

5. Emballage ou bouchon selon l'une quelconque des revendications précédentes, caractérisé en ce que lesdits moyens (30) comprennent une réduction de la force d'attraction par capillarité entre ledit produit et la région entourant lesdits moyens de purge (20) et, par conséquent, permettent à la force de gravité, s'exerçant sur ledit produit, d'être suffisante pour amener ledit produit à s'écouler loin de ladite surface extérieure (22) desdits moyens de purge, ladite réduction de la force d'attraction par capillarité étant obtenue via lesdits moyens de drainage (35).

6. Emballage ou bouchon selon l'une quelconque des revendications précédentes, caractérisé en ce que lesdits moyens (30) comprennent au moins une augmentation locale du gradient de cisaillement agissant sur ledit produit durant le drainage dudit produit à partir de ladite surface extérieure (22) desdits moyens de purge (20) et, par conséquent, réduisent la viscosité de produits ayant des propriétés d'écoulement non-newtonien, de réduction de cisaillement, ladite au moins augmentation locale du gradient de cisaillement étant obtenue par lesdits moyens de drainage (35).

7. Emballage ou bouchon selon l'une quelconque des revendications précédentes, caractérisé en ce que lesdits moyens (30) comprennent une augmentation de l'énergie superficielle résiduelle entre ladite surface extérieure (22) desdits moyens de purge (20) et la région qui entoure ladite surface extérieure desdits moyens de purge pour renforcer le drainage dudit produit loin de ladite surface extérieure desdits moyens de purge.

8. Emballage ou bouchon selon la revendication 7, caractérisé en ce que lesdits moyens (30) comportent un matériau qui a plus d'affinité avec ledit produit que ladite surface extérieure (22) desdits moyens de purge.

9. Emballage ou bouchon selon l'une quelconque des revendications 7 et 8, caractérisé en ce que lesdits moyens de purge (20) comportent une surface périphérique (32), ladite surface périphérique s'étendant à partir d'au moins ladite surface extérieure (22) desdits moyens de purge et lui étant reliés, ladite surface périphérique étant une extension parallèle de ladite surface extérieure desdits moyens de purge, et ladite surface périphérique étant plus hydrophile que ladite surface extérieure desdits moyens de purge.

10. Emballage ou bouchon selon la revendication 4, caractérisé en ce que lesdits moyens (30) comprennent un positionnement à peu près vertical desdits moyens de purge (20) et/ou de ladite surface extérieure (22) desdits moyens de purge.

11. Emballage ou bouchon selon l'une quelconque des revendications 4 à 10, caractérisé en ce que l'angle d'inclinaison α desdits moyens de purge et/ou de ladite surface extérieure desdits moyens de purge est compris entre 10° et 90°.

12. Emballage ou bouchon (10) selon l'une quelconque des revendications précédentes, caractérisé en ce que lesdits moyens de drainage (35) sont inclinés d'une manière à peu près verticale par rapport à la surface extérieure desdits moyens de purge.

13. Emballage ou bouchon selon l'une quelconque des revendications précédentes, caractérisé en ce que l'angle d'inclinaison β desdits moyens de drainage (35) par rapport à la direction horizontale est compris entre 10° et 90°.

14. Emballage ou bouchon selon l'une quelconque des revendications précédentes, caractérisé en ce que lesdits moyens de drainage (35) sont constitués d'un matériau qui est plus hydrophile que ladite surface extérieure (22) desdits moyens de purge (20).

15. Emballage ou bouchon selon l'une quelconque des revendications précédentes, caractérisé en ce que lesdits moyens de drainage (35) comprennent en outre une mèche (31) qui s'étend à partir desdits moyens de purge, vers le bas, à l'intérieur dudit emballage ou bouchon.

16. Emballage ou bouchon selon l'une quelconque des revendications précédentes, caractérisé en ce que lesdits moyens de purge (20) comportent un orifice (21) reliant l'intérieur à l'extérieur dudit emballage, et une membrane (23) recouvrant ledit orifice ou une partie de celui-ci qui permet le passage de gaz, mais empêche le passage de produits liquides.

17. Emballage ou bouchon selon l'une quelconque des revendications précédentes, caractérisé en ce que ledit produit a une viscosité comprise entre 200 cps et 5000 cps, telle que mesurée en utilisant un viscosimètre de Brookfield à 60 tpm, broche 3 et à 20°Celsius.

18. Emballage ou bouchon selon l'une quelconque des revendications précédentes, caractérisé en ce que ledit produit comprend un décolorant.

19. Emballage ou bouchon selon la revendication 18, caractérisé en ce que ledit produit comprend un décolorant peroxygénique.
